Europäisches Patentamt

European Patent Office

Office européen des brevets

(11) Veröffentlichungsnummer: **0 307 391**
**A2**

## (12) EUROPÄISCHE PATENTANMELDUNG

(21) Anmeldenummer: 88890234.3

(22) Anmeldetag: 07.09.88

(51) Int. Cl.⁴: **A 61 G 10/00**
**A 61 H 33/00**

(30) Priorität: 08.09.87 AT 2270/87

(43) Veröffentlichungstag der Anmeldung:
15.03.89 Patentblatt 89/11

(84) Benannte Vertragsstaaten:
BE CH DE FR GB IT LI NL SE

(71) Anmelder: Steiner, Josef
Kirlstrasse 7
A-3370 Ybbs (AT)

(72) Erfinder: Steiner, Josef
Kirlstrasse 7
A-3370 Ybbs (AT)

(74) Vertreter: Kliment, Peter, Dipl.-Ing. Mag.-jur.
Singerstrasse 8/3/8
A-1010 Wien (AT)

(54) Einrichtung zur homogenen Strahlungsklimatisierung eines Raumes.

(57) Einrichtung zur homogenen Strahlungsklimatisierung eines Raumes, insbesondere eines Tepidariums, mit wärmeisolierenden Wänden, wobei die dem Raum zugekehrten Seiten als Strahlungsflächen oder eine Wärmestrahlung reflektierende Flächen ausgebildet sind und Be- und Entlüftungsöffnungen vorgesehen sind. Um eine solche Einrichtung auf einfache Weise herstellen zu können, auch wenn diese im wesentlichen gekrümmte Wände aufweist, ist vorgesehen, daß die dem Raum zugekehrten Sichtflächen der Wände desselben durch sich in Längsrichtung des Raumes erstreckende schmale Platten (17), vorzugsweise aus Holz, Ton oder erhärtetem Kalkmörtel, verkleidet sind.

Fig.3

EP 0 307 391 A2

**Beschreibung**

Die Erfindung bezieht sich auf eine Einrichtung zur homogenen Strahlungsklimatisierung eines Raumes, insbesondere eines Tepidariums, mit wärmeisolierten Wänden, wobei die dem Raum zugekehrten Seiten als Strahlungsflächen oder eine Wärmestrahlung reflektierende Flächen ausgebildet sind und Be- und Entlüftungsöffnungen vorgesehen sind.

Eine solche Einrichtung wurde z.B. durch die DE-PS 29 12 196 bekannt. Bei dieser bekannten Einrichtung weist der strahlungsklimatisierte Raum die Gestalt eines Parallelepipeds auf, dessen dem Raum zugekehrten Seiten der Wände und der Decke zu einem großen Teil mit einem elektrischen Flächenheizleiter versehen sind und bei dem die Zuluftöffnung im Bereich des Bodens und die Abluftöffnung im deckennahen Bereich einer Seitenwand angeordnet ist.

Der Aufenthalt in einem solchen Raum übt auf den Menschen eine sehr günstigen Einfluß aus. So haben die Forschungen von Dr. H. Krammer und Dr. W. Ledwina ergeben, daß es durch eine Wärmebehandlung in einem solchen Raum, in dem die Temperatur der Raumluft deutlich unterhalb der Temperatur der strahlenden Flächen gehalten werden kann, auf sehr einfache Weise zu einer Entspannung der Blutgefäße kommt, sowie Unterstützung der den Blutkreislauf fördernden arteriolären Peristaltik zu beobachten.

Weiters haben Dr. H. Krammer und Dr. W. Ledwina entdeckt, daß der Blutdruck von Hypertonikern in einem solchen Raumklima binnen 20 min auf basale Werte zurückgeführt wird und daß negative Nachbilder im Auge etwa doppelt so schnell verschwinden, so daß also die Erholungszeit für eine sensorische Aufnahmefähigkeit um bis zu 50% verkürzt ist.

Dr. H,. Lueder beobachtete an einer Patientin, die starke, die Beweglichkeit einschränkende rheumatische Beschwerden aufwies, und drei Monate lang wöchentlich einmal eine Nacht in einem Tepidarium mit 36°C Strahlungstemperatur und 25°C Raumlufttemperatur geschlafen hatte, eine vegetative Gesamtumschaltung von der antiinfektiösen in die antiphlogistische Phase, wonach die rheumatischen Schmerzen vollkommen verschwanden und die Beweglichkeit der Gliedmaßen auch auf Dauer wiederhergestellt war.

Dr. H. Lueder stellte außerdem fest, daß der gesunde Mensch im Strahlungsklima mit allseitiger Strahlungstemperatur von 37°C und 25 bis 28°C Raumlufttemperatur mit wesentlich verkürztem, vielfach nur mit 4 Stunden langem Nachtschlaf auskommt. Wiederholtes Schlafen in einem solchen Strahlungsklima führt in krankhaften Fällen zu gleichmäßiger Durchblutung der Körperschale, zum Abbau von Fettpolstern und Zellulitis, zur Verjüngung der Haut am ganzen Körper, insbesondere auch am Kopf, wobei bereits ergraute Haare durch solche der früheren Haarfarbe ersetzt wurden, und in einem Fall von Epilepsie zum Verschwinden der gefürchteten Anfälle.

Aus Gründen einer möglichst gleichmäßigen Wärmebestrahlung einer im Inneren eines solchen Raumes befindlichen Person weisen solche Räume meist gekrümmte Wände auf, die sich nur relativ schwer herstellen lassen, wobei auch noch darauf bedacht genommen werden muß, daß bei der Wahl der Materialien für die Herstellung der Wände diese nach bestimmten Kriterien, u.zw. gute Wärmeabstrahlung und bzw. oder gute Wärmereflexionseigenschaften, ausgesucht werden müssen.

Bei den bekannten Lösungen wurden meist Holzwände vorgesehen und die einzelnen Teile in eine entsprechende Form gebracht, was jedoch mit einem sehr erheblichen Aufwand verbunden ist, da dabei oft auch ein Biegen von Holzbrettern erforderlich ist.

Ziel der Erfindung ist es, diese Nachteile zu vermeiden und eine Einrichtung der eingangs erwähnten Art vorzuschlagen, die sich einfach herstellen läßt.

Erfindungsgemäß wird dies dadurch erreicht, daß die dem Raum zugekehrten Sichtflächen der Wände desselben durch sich in Längsrichtung des Raumes erstreckende schmale Platten, vorzugsweise aus Holz, Ton oder erhärtetem Kalkmörtel, verkleidet sind.

Diese Platten können mit entsprechend kleinen Abmessungen hergestellt werden, wodurch auch eine bessere Ausnutzung des Rohmaterials, insbesondere bei Holz möglich ist, wobei diese Platten eben verlegt werden können, selbst wenn der Raum ausschließlich gekrümmte Wände aufweist. Die in einem solchen Falle entstehenden Kanten, da ja die Sichtseiten der Wände Vielecken mit sehr hoher Eckenzahl entsprechen. Damit ist es auch ohn weiteres möglich, in einem Spitzbogen zusammen führende Wände zu verkleiden.

Durch die Verwendung von Holz, Ton oder Kalkmörtel als Material für die Platten ergibt sich der Vorteil, daß Materialien verwendet werden, die in bauphysiologischer Hinsicht sehr günstige Eigenschaften aufweisen und sich gut als Wärmestrahler eignen.

Weiters kann vorgesehen sein, daß die die Sichtflächen bildenden Platten an ihren einander anliegenden Seitenwänden mit Nuten versehen sind, in denen von einem Heizmedium durchströmbare Leitungen, vorzugsweise Kupferrohre eingelegt sind.

Durch diese Maßnahmen wird eine sichere Halterung der das Heizmedium führenden Leitungen sichergestellt, ohne daß es dazu einer separaten Halterung bedarf. Außerdem wird dadurch auch auf einfache Weise eine gleichmäßige Erwärmung der Wände des Raumes erreicht.

In diesem Zusammenhang kann weiters vorgesehen sein, daß die Leitungen in einem Endbereich der Wände über Bögen in der angrenzenden Nut zurückgeführt sind und deren eine Enden mit einem mit dem Vorlauf verbundenen Sammler und deren andere Enden mit einem mit dem Rücklauf verbundenen Sammler nach Art eines Registers verbunden sind.

Auf diese Weise ergibt sich eine sehr einfache Montage der Leitungen, die aus bauphysiologischen Gründen vorzugsweise aus Kupfer hergestellt sind.

Grundsätzlich können auch andere Rohre, z.B. Eisenrohre, verwendet werden, doch ergibt sich bei solchen der Nachteil einer entsprechenden Beeinflussung des Magnetfeldes bzw. eine Anschirmung desselben, was für empfindliche Menschen zu ungünstigen Auswirkungen führen kann.

Zweckmäßigerweise ist vorgesehen, daß die mit dem Vor- und Rücklauf verbundenen Sammler der Leitungen in einem stirnseitig an den strahlungsklimatisierten Raum anschließenden und von diesem durch eine Wand getrennten Raum angeordnet sind, in dem gegebenenfalls auch eine Einrichtung zur Erwärmung des Heizmediums angeordnet ist.

Dadurch ergibt sich ein sehr kompakter Aufbau der Einrichtung, wobei sich auch noch der Vorteil ergibt, daß die die beiden Räume trennende Wand in Bezug auf den strahlungsklimatisierten Raum gleichzeitig als Strahlungswand wirkt und auf diese Weise die bei der Erwärmung des Heizmediums anfallende Verlustwärme zum Teil auch dem zu klimatisierenden Raum zuführt.

Die Erfindung wird nun anhand der Zeichnung näher erläutert. Dabei zeigen:

Fig. 1 einen Querschnitt durch eine erfindungsgemäße Einrichtung,

Fig. 2 einen Längsschnitt durch die Einrichtung gemäß der Fig. 1,

Fig. 3 eine schematische Darstellung des Aufbaues der Innenauskleidung einer erfindungsgemäßen Einrichtung, und

Fig. 4 ein Detail der Auskleidung.

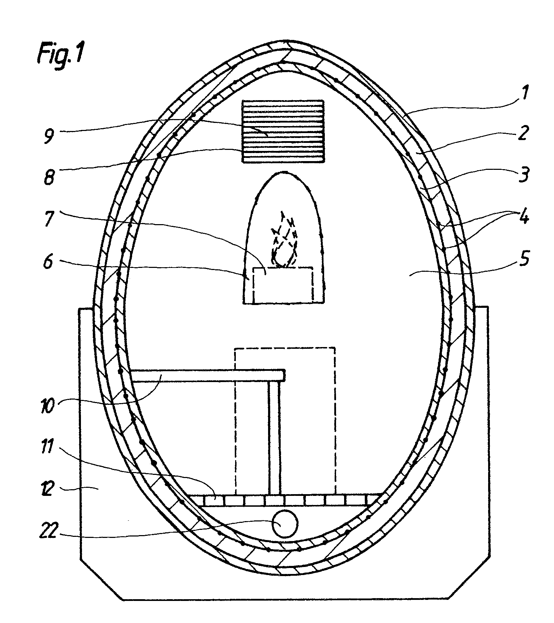

Wie aus der Fig. 1 zu ersehen ist, weist die erfindungsgemäße Einrichtung einen im wesentlichen eiförmigen Querschnitt auf. Diese Einrichtung weist eine dichte und die entsprechende Festigkeit sicherstellende Außenschale 1 auf, die an ihrer Innenseite mit einer wärmedämmenden Schichte 2 beschichtet ist, die gegebenenfalls eine reflektierende Folie umfassen kann. Diese wärmedämmende Schichte 2 ist gegen das Innere der Einrichtung zu mittels einer Auskleidung 3 abgedeckt, wobei eine Heizeinrichtung 4 zwischen der wärmedämmenden Schichte 2 und der Auskleidung 4 oder im Bereich derselben angeordnet ist.

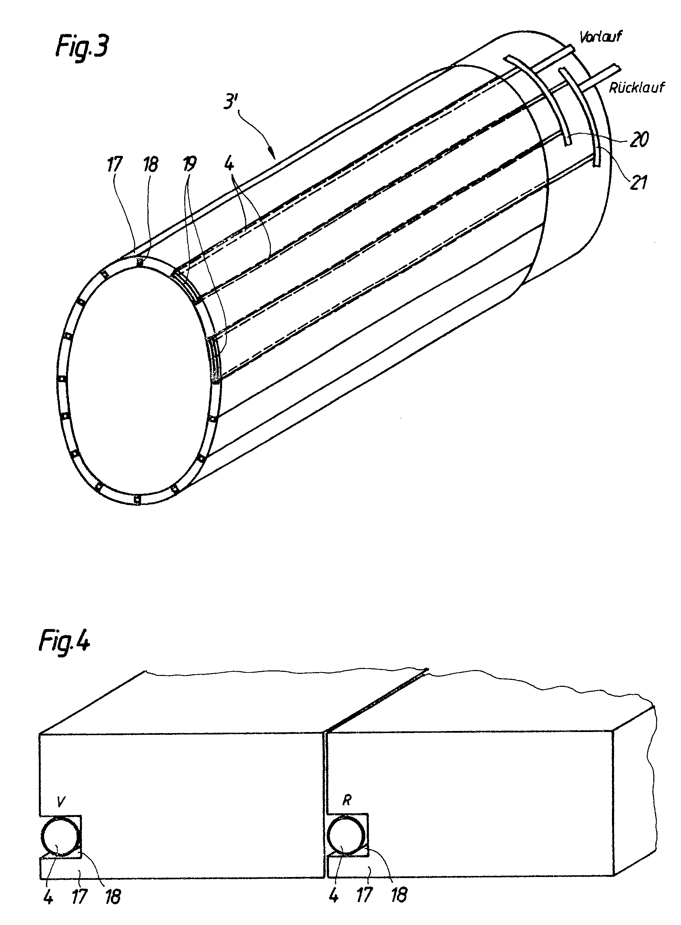

Bei der Ausführungsform gemäß der Fig. 1 ist die Heizeinrichtung, die durch von einem Heizmedium, wie z.B. Warmwasser durchströmte Rohre 4 gebildet sein kann, zwischen der wärmedämmenden Schichte 2 und der Auskleidung 3 angeordnet, wogegen die Fig. 3 eine Ausführungsform darstellt, bei der die Heizeinrichtung bzw. deren Rohre 4 im Bereich der Auskleidung 3 angeordnet ist.

In jedem Falle kommt es beim Betrieb der Einrichtung zu einer Erwärmung der Auskleidung 3, deren dem Inneren des Raumes 5 zugekehrte Seite als Strahlungsfläche dient und die Wärme in den zu klimatisierenden Raum abgibt.

In den klimatisierten Raum 5 ist eine weitere Wärmequelle 6 angeordnet, die im wesentlichen als Hohlkörper ausgebildet ist, in dessen Innerem eine Heizeinrichtung 7 angeordnet ist. Um allenfalls störende Magnetfelder im Inneren des klimatisierten Raumes 5 zu vermeiden, ist diese Heizeinrichtung 7 vorzugsweise als Brenner ausgebildet, wobei nicht dargestellte Leitungen für die Luftzufuhr und die Abfuhr der Abgase, sowie für die Zufuhr des Brennstoffes vorgesehen sind.

Für die Belüftung des Raumes 5 ist an einer Stirnwand 23 desselben in deren oberem Bereich eine Belüftungsöffnung 8 vorgesehen, in der ein gaselektronisches Gebläse 9 angeordnet ist, das geräuschlos für eine Zwangsbelüftung sorgt. Gleichzeitig wird dadurch die zugeführte Luft von Staubpartikeln gereinigt und mit Ionen angereichert.

Weiters ist in dem Raum 5 noch eine Sitz- oder Liegefläche 10 sowie ein Fußboden 11 angeordnet, unter dem eine Entlüftungsöffnung 22 in der einen Stirnwand des Raumes 5 angeordnet ist.

Um einen sicheren Stand der Einrichtung zu gewährleisten, sind zumindest zwei Rippen 12 an der Außenschale 1 befestigt, die eine ebene Aufstandsfläche aufweisen.

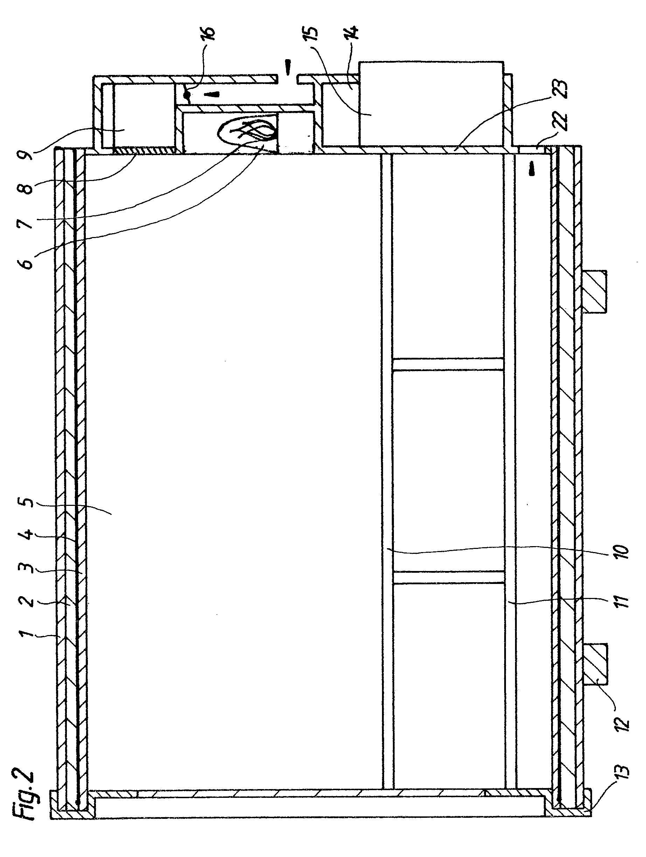

Wie aus Fig. 2 zu ersehen ist, weist die Einrichtung an der einen Stirnseite eine Abdeckung 13 auf, die einen Zutrittsquerschnitt zum Raum 5 aufweist, der gegebenenfalls verschließbar ist.

An der zweiten Stirnseite des Raumes 5 grenzt ein weiterer Raum 14 an, in dem sich ein Boiler 15 befindet, an dem die durch die Rohre 4 gebildete Heizeinrichtung angeschlossen ist. Weiters befindet sich in diesem Raum 14 eine den Zustrom der Frischluft zum Raum 5 steuernde Luftregelklappe 16.

Die Rohre 4 der Heizeinrichtung bestehen vorzugsweise aus Kupfer, da sich dieses Material durch eine gute Wärmeleitfähigkeit auszeichnet und das natürliche Erdmagnetfeld nur wenig beeinflußt.

Die Fig. 3 und 4 zeigen eine Variante einer Auskleidung 3'. Diese besteht aus sich in Längsrichtung des Raumes 5 erstreckenden, relativ schmalen Platten 17, die an deren aneinander anliegenden Seitenflächen mit Nuten 18 versehen sind. In diesen Nuten 18 sind die die Heizeinrichtung bildenden Rohre 4 eingelegt, von denen je zwei einander benachbarte Rohre im Bereich ihrer einen Enden über Bögen 19 miteinander verbunden sind. Die zweiten Enden dieser, in den Nuten 18 eingelegten Rohre 4 sind mit einer Vorlauf- bzw. Rücklaufleitung 20, 21 zu einem Register verbunden.

Dabei befinden sich die Vorlauf- bzw. Rücklaufleitungen 20, 21 zweckmäßigerweise in dem an den zu klimatisierenden Raum 5 anschließenden Raum 14, der von dem ersteren auch die Wand 23 getrennt ist.

Die Auskleidung besteht zweckmäßigerweise aus Holz-oder Tonplatten, da sich diese Materialien gut zur Abstrahlung von Wärme eignen und diese weitgehend homogen abstrahlen. Außerdem ermöglichen diese Materialien die Aufrechterhaltung einer relativ hohen Ionenkonzentration in der Luft, wodurch sich günstige physiologische Effekte ergeben. Die Stärke dieser Platten kann relativ gering gewählt werden. So hat sich gezeigt, daß eine Dicke von ca. 30 bis 40 mm durchaus ausreichend ist.

Grundsätzlich ist es auch möglich, die Außenschale 1 samt der Isolierschichte und der Auskleidung in Richtung deren Längsachse zu teilen, sodaß der obere Teil abgehoben oder aufgeschwenkt werden kann. Dabei ist es lediglich erforderlich, die Heizeinrichtung bzw. deren Rohre 4 entsprechend einer oder zwei Heizmediumquellen anzuschließen. In einem solchen Falle kann der Raum 5 durch zwei durchgehende Stirnwände abgeschlossen werden, da dann ein Zutritt bei geöffnetem Oberteil möglich ist.

**Patentansprüche**

1. Einrichtung zur homogenen Strahlungsklimatisierung eines Raumes, insbesondere eines Tepidariums, mit wärmeisolierenden Wänden, wobei die dem Raum zugekehrten Seiten als Strahlungsflächen oder eine Wärmestrahlung reflektierende Flächen ausgebildet sind und Be- und Entlüftungsöffnungen vorgesehen sind, dadurch gekennzeichnet, daß die dem Raum zugekehrten Sichtflächen der Wände desselben durch sich in Längsrichtung des Raumes erstreckende schmale Platten (17), vorzugsweise aus Holz, Ton oder erhärtetem Kalkmörtel, verkleidet sind.

2. Einrichtung nach Anspruch 1, dadurch gekennzeichnet, daß die die Sichtflächen bildenden Platten (17) an ihren einander anliegenden Seitenwänden mit Nuten (18) versehen sind, in denen von einem Heizmedium durchströmbare Leitungen, vorzugsweise Kupferrohre (4) eingelegt sind.

3. Einrichtung nach Anspruch 2, dadurch gekennzeichnet, daß die Leitungen in einem Endbereich der Wände über Bögen (19) in der angrenzenden Nut (18) zurückgeführt sind und deren eine Enden mit einem mit dem Vorlauf (20) verbundenen Sammler und deren andere Enden mit einem mit dem Rücklauf (21) verbundenen Sammler nach Art eines Registers verbunden sind.

4. Einrichtung nach Anspruch 2 oder 3, dadurch gekennzeichnet, daß die mit dem Vor- und Rücklauf (20, 21) verbundenen Sammler der Leitungen in einem stirnseitig an den strahlungsklimatisierten Raum (5) anschließenden und von diesem durch eine Wand (23) getrennten Raum (14) angeordnet sind, in dem gegebenenfalls auch eine Einrichtung (25) zur Erwärmung des Heizmediums angeordnet ist.

**Fig.1**

Fig.2

Fig.3

3'

17  18  19  4

Vorlauf

Rücklauf

20

21

Fig.4

V

R

4  17  18

4  17  18